# EUROPEAN PATENT APPLICATION

(11) **EP 4 736 765 A1**
(43) Date of publication of application: **06.05.2026**
(21) Application number: 24831793.5
(22) Date of filing: 19.06.2024
(51) Int. Cl.: A61B 5/291, A61B 5/256

(54) **ELECTROENCEPHALOGRAM MEASUREMENT DEVICE**

(30) Priority: 27.06.2023 JP 2023104854
(71) Applicant: Sumitomo Bakelite Co.Ltd., Shinagawa-ku Tokyo 140-0002 (JP)
(72) Inventor: ISHIKAWA, Eiji, Tokyo 140-0002 (JP)
(74) Representative: Ter Meer Steinmeister & Partner
(86) International application number: PCT/JP2024/022233
(87) International publication number: WO 2025/004931

(57) **Abstract**

An electroencephalogram measurement device (10) includes a support (110), an electroencephalogram electrode member (120), and a tube (151). The support (110) is mountable on a head. The electroencephalogram electrode member (120) is held by the support (110). The tube (151) passes through a third through hole (121) provided in the electroencephalogram electrode member (120). The tube (151) may be insertable into and removable from the third through hole (121) of the electroencephalogram electrode member (120).

## Description

### TECHNICAL FIELD

The present invention relates to an electroencephalogram measurement device.

### BACKGROUND ART

In electroencephalogram measurement, electrodes are placed on a head to conduct electrical measurements.

Patent Document 1 discloses a probe for electroencephalogram measurement, in which a tip capable of allowing an electrolytic solution to bleed out is brought into contact with a skin of the head.

### RELATED DOCUMENT

### PATENT DOCUMENT

[Patent Document 1] Japanese Unexamined Patent Publication No. H1-236036

### SUMMARY OF THE INVENTION

### TECHNICAL PROBLEM

However, in the technique disclosed in Patent Document 1, since a half cell is configured by the electrode and the electrolytic solution in the tip for performing the measurement, the structure of the probe is complicated.

The present invention provides a simple electroencephalogram measurement device capable of supplying a liquid to a scalp and performing measurement.

### SOLUTION TO PROBLEM

According to one aspect of the present invention, the following electroencephalogram measurement device is provided.

1. An electroencephalogram measurement device including:
   a support which is mountable on a head;
   an electroencephalogram electrode member which is held by the support; and
   a tube which passes through a through hole provided in the electroencephalogram electrode member.
2. The electroencephalogram measurement device according to 1.,
   in which the electroencephalogram electrode member includes a base and one or more protruding portions provided in the base, and
   the through hole is provided in the base.
3. The electroencephalogram measurement device according to 2.,
   in which the tube penetrates the base.
4. The electroencephalogram measurement device according to 3.,
   in which a protrusion height of the tube from the base is smaller than a protrusion height of the protruding portion from the base.
5. The electroencephalogram measurement device according to any one of 1. to 4.,
   in which the tube is insertable into and removable from the through hole of the electroencephalogram electrode member.
6. The electroencephalogram measurement device according to any one of 1. to 5., further including:
   an injection member for injecting a liquid into the tube.

### ADVANTAGEOUS EFFECTS OF INVENTION

According to the present invention, it is possible to provide a simple electroencephalogram measurement device capable of supplying a liquid to a scalp and performing measurement.

### BRIEF DESCRIPTION OF THE DRAWINGS

FIG. 1 is a view showing a partial cross section of the electroencephalogram measurement device according to the embodiment.
FIG. 2 is a perspective view showing the electroencephalogram measurement device according to the embodiment.
FIG. 3 is a perspective view showing the electroencephalogram measurement device according to the embodiment.
FIG. 4 is a view showing a state in which a support is mounted on a head of a person.
FIG. 5 is a view showing an inside state of the support.
FIG. 6 is a view showing a state in which the support is pressed against a scalp of the head.
FIG. 7 is a view showing a way of moving a holding member.
FIG. 8 is a view showing a state in which a tube is attached to the support.
FIG. 9 is a cross-sectional view showing a state of an electroencephalogram electrode member during measurement and a periphery thereof.
FIG. 10 is a view showing a partial cross section of the electroencephalogram measurement device in a state in which the holding member and the electroencephalogram electrode member are detached from the support.
FIG. 11 is a view showing a structure of the electroencephalogram electrode member on a side facing the head.
FIG. 12 is a side view of the electroencephalogram electrode member.
FIG. 13 is a cross-sectional view taken along a line A-A of FIG. 11.
FIG. 14 is a view showing a relationship between a protruding portion and a tube.

### DESCRIPTION OF EMBODIMENTS

Hereinafter, embodiments of the present invention will be described with reference to the drawings. In all drawings, the same constituents are designated by the same reference numerals, and description thereof will not be repeated.

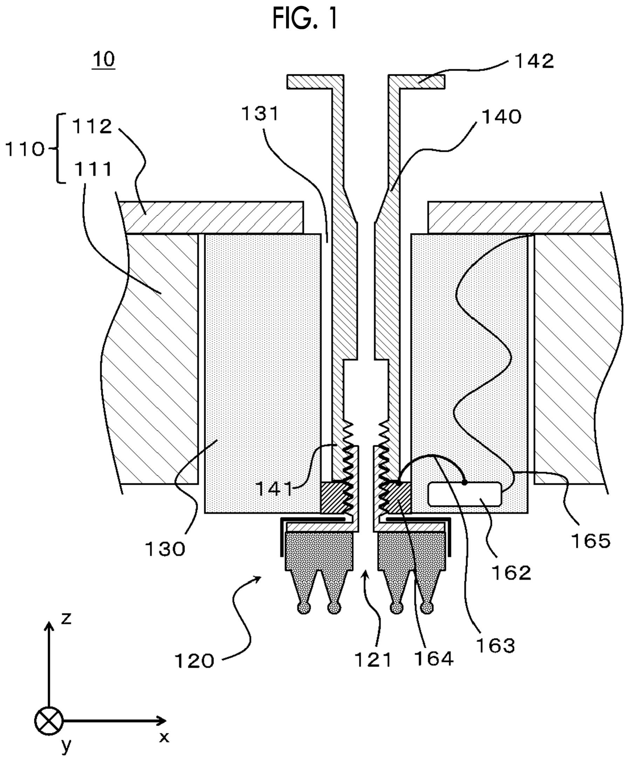

FIG. 1 is a view showing a partial cross section of an electroencephalogram measurement device 10 according to an embodiment. The electroencephalogram measurement device 10 includes a support 110 and an electroencephalogram electrode member 120. The support 110 is mountable on a head. The electroencephalogram electrode member 120 is held by the support 110.

The electroencephalogram can be measured by bringing the electroencephalogram electrode member 120 of the electroencephalogram measurement device 10 into contact with the head.

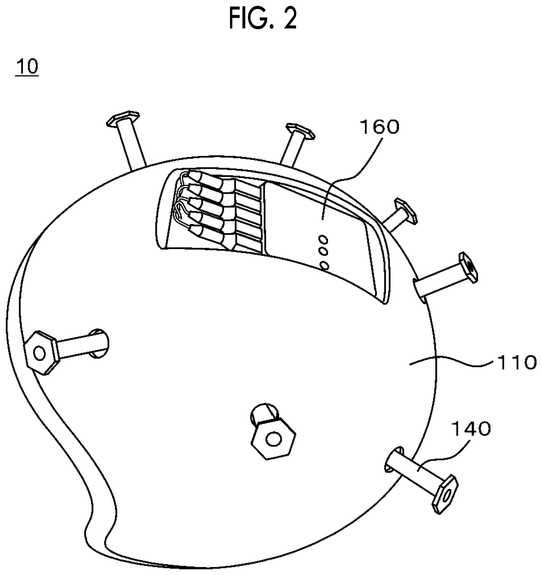

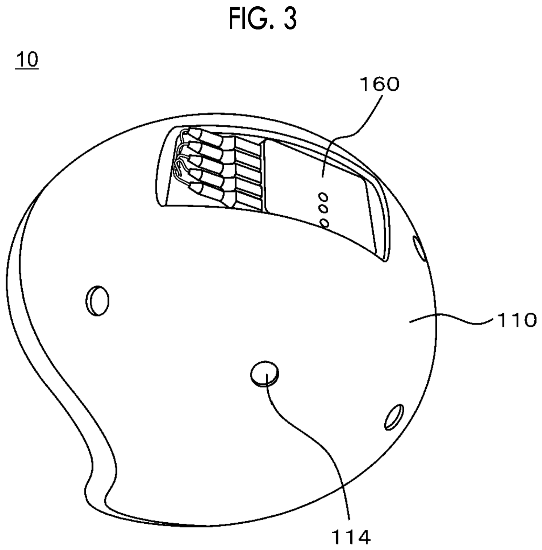

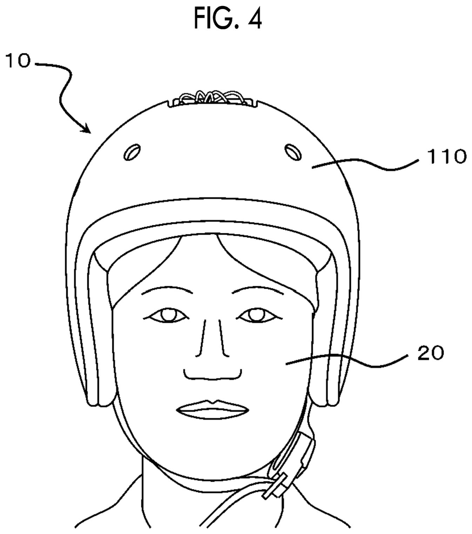

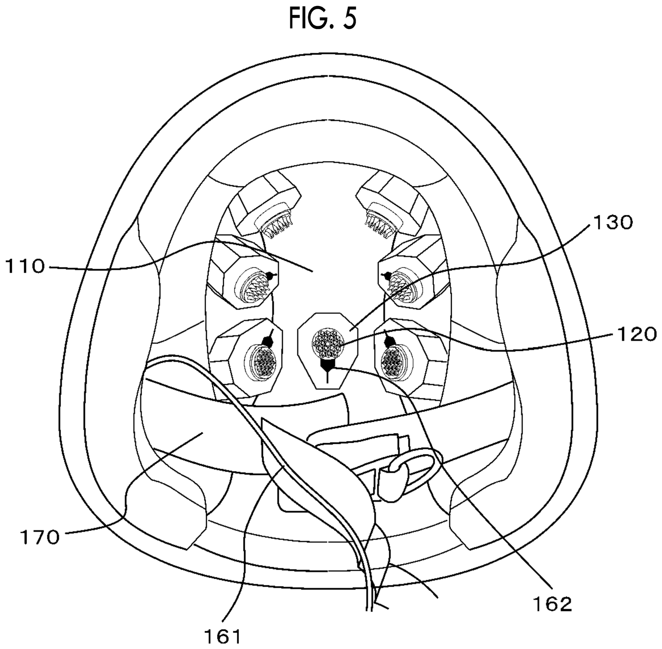

FIGS. 2 and 3 are perspective views showing the electroencephalogram measurement device 10 according to the present embodiment. FIG. 4 is a view showing a state in which the support 110 is mounted on a head 20 of a person. FIG. 5 is a view showing a state of an inside (a side into which the head 20 is inserted) of the support 110. In the example of FIGS. 2 to 5, the support 110 has a helmet shape. In a case where the support 110 has a helmet shape, the support 110 has a recessed portion into which the head 20 is inserted. The electroencephalogram measurement device 10 is configured such that, in a state in which the support 110 having a helmet shape is mounted on the head 20, one or more electroencephalogram electrode members 120 are in contact with the head 20. The electroencephalogram measurement device 10 may include a belt 170 for fixing the support 110 to the head 20 as shown in FIG. 5.

Hereinafter, a side of the support 110 facing the head 20 is referred to as the inside of the support 110, and a side opposite to the inside is referred to as an outside of the support 110. In FIG. 1, a direction from the inside to the outside of the support 110 is defined as a z direction. An x direction, a y direction, and the z direction are orthogonal to each other. The z direction is substantially a normal direction of a scalp 22. The x direction, the y direction, and the z direction may be defined as different directions for each of the electroencephalogram electrode members 120 in the electroencephalogram measurement device 10.

The holding member 140, which will be described in detail later, may be attachable to and detachable from the support 110. FIG. 2 shows a state in which the holding member 140 is attached to the support 110, and FIGS. 3 and 4 show a state in which the holding member 140 is detached from the support 110.

In the example of FIGS. 2 to 5, the support 110 holds the plurality of electroencephalogram electrode members 120. In a state in which the support 110 is mounted on the head 20, each of the electroencephalogram electrode members 120 can be brought into contact with a predetermined position of the head 20. Electroencephalogram is measured by the plurality of electroencephalogram electrode members 120. For example, the support 110 can hold seven electroencephalogram electrode members 120. Positions of the seven electroencephalogram electrode members 120 may correspond to positions of F3, F4, C3, C4, P3, Pz, and P4, in an international 10-20 electrode placement method. The number and the position of the electroencephalogram electrode members 120 provided in the support 110 are not particularly limited, and can be set according to the use or the like.

In the example of FIG. 1, the electroencephalogram electrode member 120 is provided with a third through hole 121. The third through hole 121 is a hole for supplying a liquid to the head 20. By providing the third through hole 121 in the electroencephalogram electrode member 120, the liquid can be easily injected into the scalp. For example, in a case where an auxiliary liquid containing an electrolyte is supplied to the head 20 before measuring the electroencephalogram, the electrical contact between the scalp and the electroencephalogram electrode member 120 can be improved. The electroencephalogram electrode member 120 and the method of measuring the electroencephalogram will be described in detail below.

In the example of FIG. 1, the electroencephalogram measurement device 10 further includes an elastic member 130 and a holding member 140. The elastic member 130 is elastically deformable. The elastic member 130 is provided with a first through hole 131. The holding member 140 passes through the first through hole 131, in which one end 141 holds the electroencephalogram electrode member 120 and the other end 142 is exposed to the outside of the support 110. The electroencephalogram electrode member 120 is held by the support 110 through the elastic member 130.

The support 110 has, for example, a shape determined based on an average head shape. However, the head shape varies greatly among individuals, and an element which absorbs the difference is required. In the electroencephalogram measurement device 10 in which the electroencephalogram electrode member 120 is held by the support 110 through the elastic member 130, the elastic member 130 is elastically deformed in a state in which the support 110 is mounted on the head 20. In this way, even in a case where there is an individual difference (unevenness or an angle of a surface) in the head shape, the electroencephalogram electrode member 120 can be stably brought into contact with the scalp to measure the electroencephalogram.

In addition, by combining the elastic member 130 and the holding member 140 in the electroencephalogram measurement device 10, the electroencephalogram electrode member 120 can be moved by using the holding member 140. That is, the hair can be combed by the electroencephalogram electrode member 120, and the electroencephalogram electrode can be stably brought into contact with the scalp.

In an example of the electroencephalogram measurement device 10, the holding member 140 is attachable to and detachable from the electroencephalogram electrode member 120. In this way, a protruding portion from the support 110 can be eliminated during the measurement, and the center of gravity in a state of being mounted on the head 20 can be stabilized. In addition, the subject to be measured can lie down in a state of being mounted with the support 110, and noise derived from body movement or the like can be reduced. In addition, the electroencephalogram measurement may be performed while the subject to be measured moves around.

In addition, the electroencephalogram electrode member 120 is attachable to and detachable from the support 110. In this way, the electroencephalogram electrode member 120 can be replaced as necessary, or different types of electroencephalogram electrode members 120 can be used for each measurement.

A method of using the electroencephalogram measurement device 10 will be described below. First, as shown in FIG. 1, the support 110 in a state of having the electroencephalogram electrode member 120 and the holding member 140 attached is mounted on the head 20. However, the holding member 140 may be attached to the electroencephalogram electrode member 120 after the support 110 is mounted on the head 20.

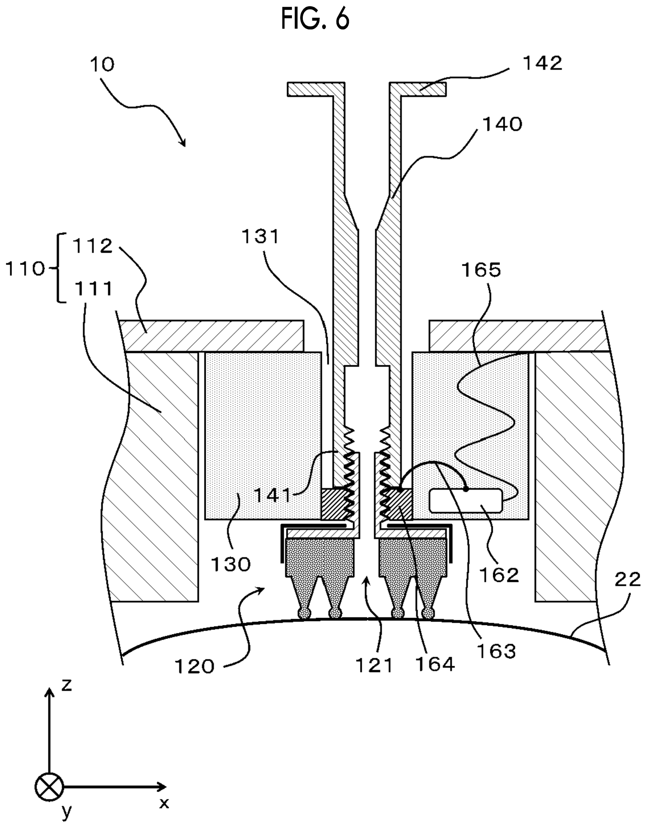

FIG. 6 is a view showing a state in which the support 110 is pressed against the scalp 22 of the head 20. FIG. 6 is a partial cross-sectional view of the electroencephalogram measurement device 10. The cross section shown in FIG. 6 corresponds to the cross section shown in FIG. 1. In a state in which the support 110 is pressed against the scalp 22 of the head 20, the elastic member 130 is contracted as compared with a state before the pressing. In a case where the support 110 is mounted on the head 20, the electroencephalogram electrode member 120 comes into contact with the scalp 22. The elastic member 130 is deformed according to the position or the angle of the scalp 22 with respect to the support 110. In addition, the electroencephalogram electrode member 120 is pressed against the scalp 22 with a force corresponding to elasticity of the elastic member 130. That is, the position and the angle of a distal end of the electroencephalogram electrode member 120 with respect to the support 110 can be changed to follow the shape of the head 20 by the contraction of the elastic member 130.

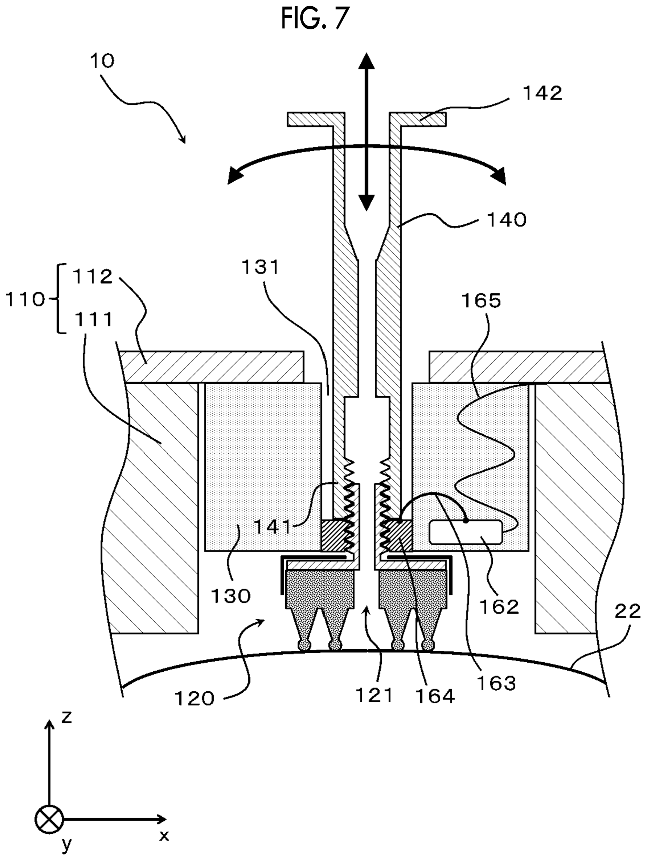

FIG. 7 is a view showing a way of moving the holding member 140. FIG. 7 is a partial cross-sectional view of the electroencephalogram measurement device 10. The cross section shown in FIG. 7 corresponds to the cross section shown in FIG. 1. In a state in which the holding member 140 holds the electroencephalogram electrode member 120, an angle of the holding member 140 with respect to the support 110 is variable. In addition, a length of a portion of the holding member 140, which is exposed to the outside from the support 110, is variable according to a degree of contraction of the elastic member 130. After the support 110 is mounted on the head 20, for example, the operator of the electroencephalogram measurement device 10 moves the holding member 140 with the support 110 as a reference.

Specifically, the operator can move the holding member 140 to change the angle of the holding member 140 with respect to the support 110, that is, with respect to the head 20. In particular, it is effective to move the holding member 140 such that the other end 142 of the holding member 140 revolves around the electroencephalogram electrode member 120 as a fulcrum. In addition, the operator can push and pull the holding member 140 with respect to the support 110, that is, the head 20. With these movements of the holding member 140, the hair of the scalp 22 can be combed, and a contact state between the electroencephalogram electrode member 120 and the scalp 22 can be improved.

Next, the operator attaches a tube to the support 110. The electroencephalogram measurement device 10 according to the present embodiment further includes a tube which passes through the third through hole 121 provided in the electroencephalogram electrode member 120. The tube is insertable into and removable from the third through hole 121 of the electroencephalogram electrode member 120.

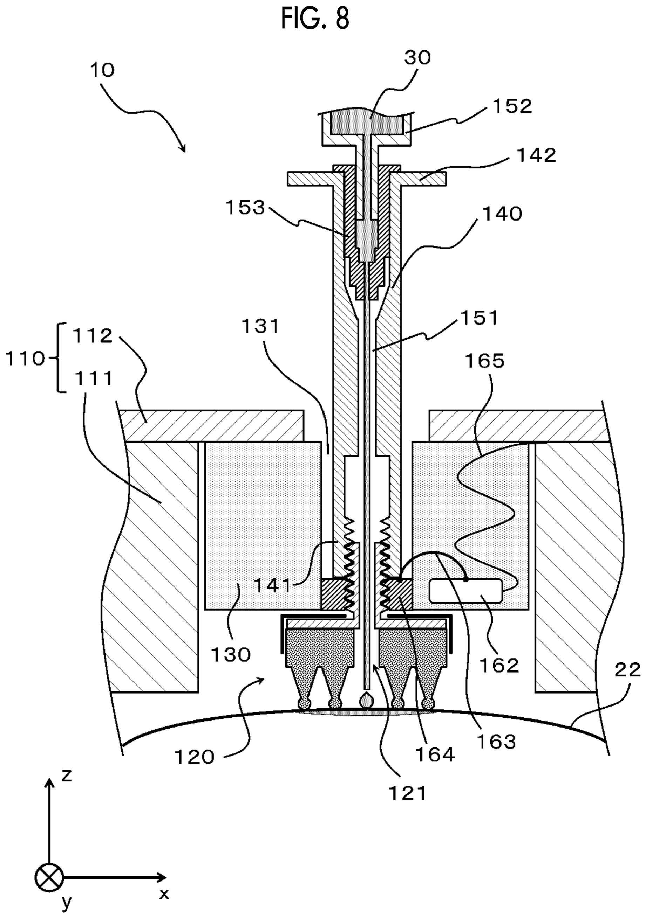

FIG. 8 is a view showing a state in which a tube 151 is attached to the support 110. FIG. 8 is a partial cross-sectional view of the electroencephalogram measurement device 10. The cross section shown in FIG. 8 corresponds to the cross section shown in FIG. 1. In addition, FIG. 6 can be said to show the support 110 in a state in which the tube 151 is detached. In the example of FIG. 8, the electroencephalogram measurement device 10 further includes an injection member 152 for injecting a liquid 30 into the tube 151. In addition, in the example of FIG. 8, the electroencephalogram measurement device 10 further includes a connecting member 153. The connecting member 153 is a connecting member for connecting the tube 151 to the injection member 152.

In a case where the operator attaches the tube 151 to the support 110, an auxiliary liquid is injected into the tube 151 to supply the auxiliary liquid to the scalp 22, and the scalp 22 is wet as shown in FIG. 8. The auxiliary liquid is an example of the liquid 30. The auxiliary liquid is not particularly limited as long as the electrical resistance between the electroencephalogram electrode member 120 and the scalp 22 can be reduced, and for example, contains an electrolyte. However, it is also possible to perform the electroencephalogram measurement without supplying the auxiliary liquid using the electroencephalogram measurement device 10.

Next, the operator detaches the holding member 140 from the support 110, and starts the electroencephalogram measurement.

According to the present embodiment, the electroencephalogram measurement device 10 includes the electroencephalogram electrode member 120 having the third through hole 121 and the tube 151 passing through the third through hole 121. Therefore, the auxiliary liquid can be efficiently supplied to a region where the electroencephalogram electrode member 120 and the scalp 22 are in contact with each other.

In addition, the auxiliary liquid can be supplied immediately before the measurement after the electroencephalogram electrode member 120 is brought into contact with the scalp 22. Therefore, a liquid having a low viscosity can be used as an auxiliary agent. For example, in a case where the auxiliary agent is applied to the electroencephalogram electrode member before the electroencephalogram electrode member is placed on the head, there is a concern that the auxiliary agent may flow or evaporate before the measurement is started, and thus a paste-like or gel-like auxiliary agent is required. In a case in which the paste-like auxiliary agent is used, since a viscosity of the auxiliary agent is very high, it is necessary to manually comb the hair and apply the auxiliary agent, which is timeconsuming. In addition, in a case where the gel-like auxiliary agent is used, the gel-like auxiliary agent is difficult to reach the scalp due to the influence of the hair, and there is a problem in that the electrode is short-circuited due to the liquid dripping in a case where the gel-like auxiliary agent is excessively applied. On the other hand, in a case where the auxiliary liquid can be supplied immediately before the measurement, there is no such concern. With the electroencephalogram measurement device 10 according to the present embodiment, the holding member 140 can be operated in advance to comb the hair and inject a small amount of the auxiliary liquid near the scalp. In addition, by using the auxiliary liquid having a low viscosity equivalent to that of water, the influence of the hair is further reduced, and thus the contact resistance can be significantly reduced with a small amount of liquid.

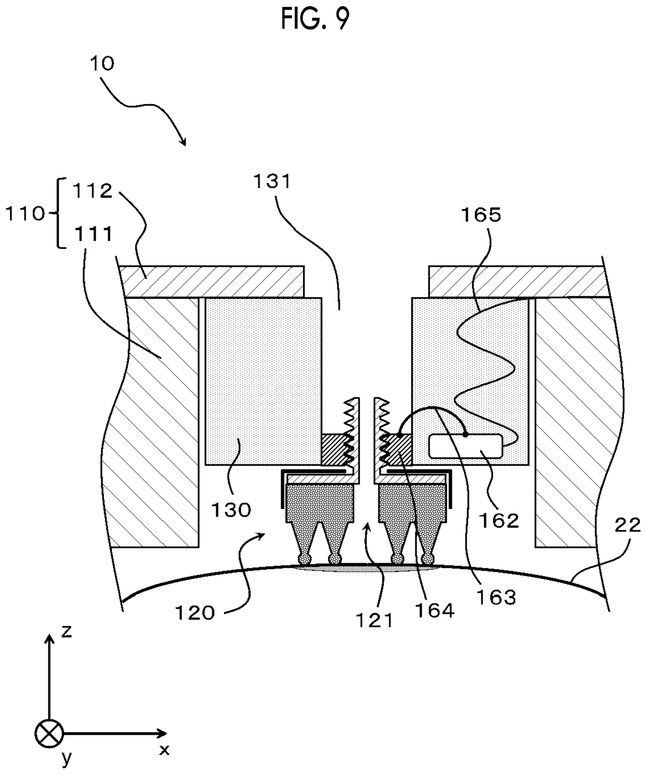

FIG. 9 is a cross-sectional view showing a state of the electroencephalogram electrode member 120 during measurement and a periphery thereof. The cross section shown in FIG. 9 corresponds to the cross section shown in FIG. 1. The electroencephalogram measurement can be performed in a state in which the tube 151 and the holding member 140 are detached from the support 110.

Each component of the electroencephalogram measurement device 10 will be described in detail below.

The support 110 has a shape capable of covering at least a part of the head 20. In the present embodiment, the support 110 includes a substrate 111 and a covering member 112. The substrate 111 is located on the head 20 side in a state in which the support 110 is mounted on the head 20. The covering member 112 is located on a side opposite to the head 20 side in a state in which the support 110 is mounted on the head 20.

The substrate 111 is formed of, for example, expanded polystyrene. The covering member 112 is formed of, for example, a resin. The covering member 112 is harder than the substrate 111, and can protect the head 20. However, the support 110 may not include the covering member 112.

Here, it is sufficient that the support 110 is mountable on the head 20, and the support 110 may be formed of, for example, cloth or rubber. The support 110 may be, for example, helmetshaped, hat-shaped, or band-shaped.

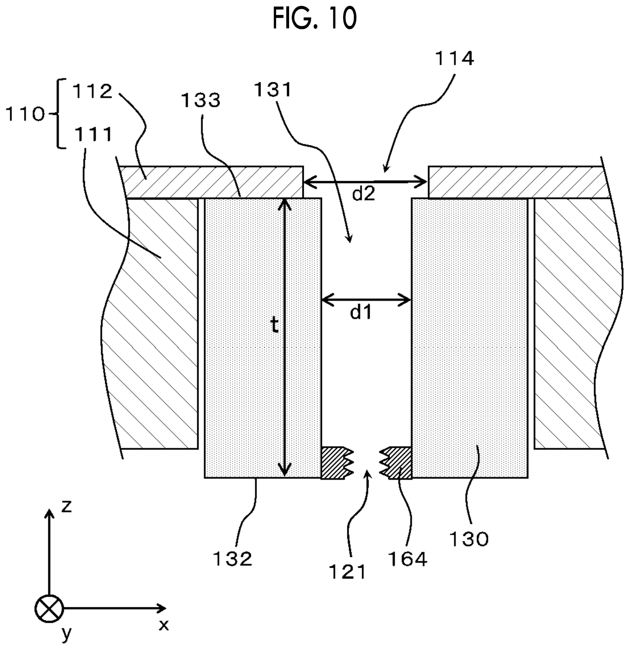

FIG. 10 is a view showing a partial cross section of the electroencephalogram measurement device 10 in a state in which the holding member 140 and the electroencephalogram electrode member 120 are detached from the support 110. The cross section shown in FIG. 10 corresponds to the cross section shown in FIG. 1. In FIG. 10, a wiring line 163, a circuit 162, and a wiring line 165, which will be described later, are not shown. The elastic member 130 is accommodated in a hole provided in the support 110. An outer shape and a size of the elastic member 130 substantially match an inner shape and a size of the hole provided in the support 110, and the elastic member 130 is fitted into the hole of the support 110. In a state in which the support 110 is not mounted on the head 20, the elastic member 130 may fill an entire portion of the hole provided in the support 110, other than the first through hole 131. In addition, as shown in FIG. 10, a part of the elastic member 130 may protrude from the hole of the support 110. A second through hole 114 is provided in the covering member 112. The first through hole 131 provided in the elastic member 130 communicates with the second through hole 114 provided in the covering member 112. For example, as shown in FIG. 1, the holding member 140 holds the electroencephalogram electrode member 120 in a state of passing through the first through hole 131 and the second through hole 114.

Here, it is preferable that a hole size d2 of the second through hole 114 is larger than a hole size d1 of the first through hole 131. By increasing the hole size d2 of the second through hole 114 in this way, the holding member 140 can be inclined more with respect to the support 110.

The elastic member 130 consists of an elastic material. The elastic material consists of, for example, one or more selected from a urethane sponge, a polyethylene sponge, a polypropylene sponge, or a silicone rubber sponge. The elastic material may be a foam body, and examples of the foam body include a low-rebound sponge and a low-rebound elastic foam.

The elastic member 130 can have a configuration which does not include a spring. In a case where a spring is used, a rebound force of the spring increases in proportion to a deformation amount of the spring. Therefore, in a case where the deformation amount is large, an excessive rebound force is generated, and the subject to be measured is likely to feel pain. On the other hand, in a case where the elastic material of the foam body is used, there is a displacement amount range in which the rebound force does not increase (proportionally) with an increase in the deformation amount. By configuring the elastic member 130 to be used in such a displacement amount range, an appropriate rebound force can be obtained even in a case where the deformation amount varies depending on the position of the electroencephalogram electrode member 120.

A hardness H of the elastic material, which is measured by a JIS K 6400-2 ·A method, is, for example, 30 N or more and 200 N or less. From the viewpoint of further reducing a burden on the subject to be measured, the hardness H is preferably 100 N or less. In addition, from the viewpoint of more stably pressing the electroencephalogram electrode member 120 against the scalp 22, the hardness H is preferably 50 N or more.

A thickness t of the elastic member 130 is, for example, 10 mm or more and 100 mm or less in a state in which the support 110 is not mounted on the head 20. Here, the thickness t is a thickness of the elastic member 130 in a direction perpendicular to a surface 132 facing the head 20. The elastic member 130 is fixed to the support 110 at one end, and the thickness t of the elastic member 130 is variable according to a force received in the thickness direction. Specifically, the elastic member 130 is fixed to the support 110 at a surface 133 on a side opposite to the surface 132. The thickness t of the elastic member 130 is preferably 20 mm or more and 60 mm or less in a state in which the support 110 is not mounted on the head 20.

An area of the surface 132 of the elastic member 130, facing the head, is, for example, 3 cm² or more and 25 cm² or less. A shape of the surface 132 is not particularly limited. Examples of the shape of the surface 132 include a circular shape, a quadrangular shape, an oval shape, and an elliptical shape.

A conductive portion 164 is fixed inside the first through hole 131 of the elastic member 130. The conductive portion 164 is, for example, a metal. The conductive portion 164 is provided with a through hole, and a screw groove for fixing the electroencephalogram electrode member 120 is provided inside the through hole.

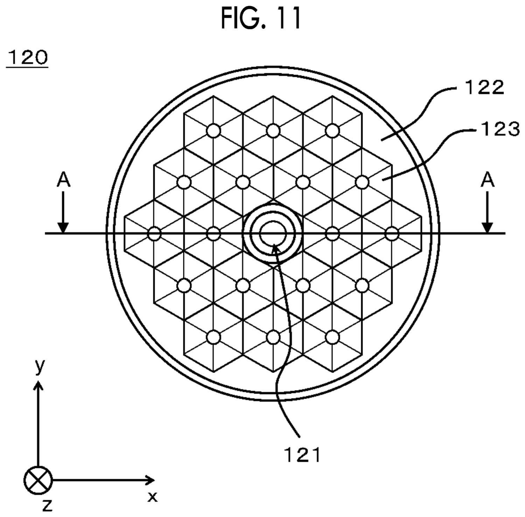

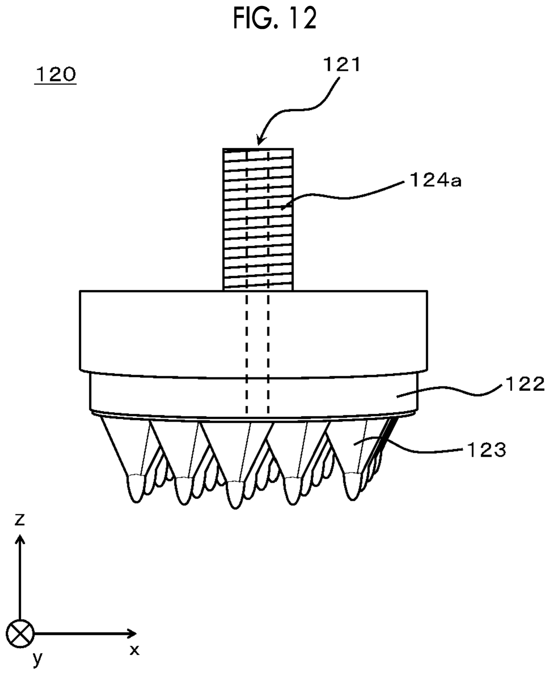

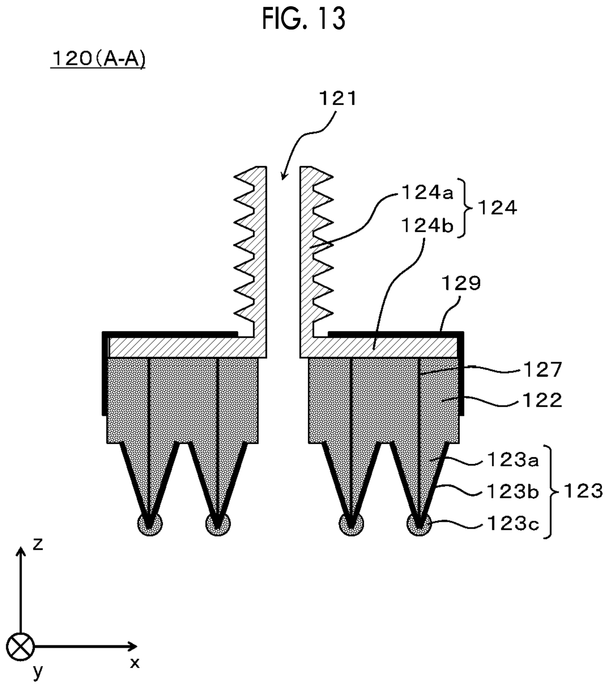

FIG. 11 is a view (bottom view) showing a structure of the electroencephalogram electrode member 120 on a side facing the head 20. FIG. 12 is a side view of the electroencephalogram electrode member 120. FIG. 13 is a cross-sectional view taken along a line A-A of FIG. 11. The electroencephalogram electrode member 120 includes a base 122 and one or more protruding portions 123 provided on the base 122. The third through hole 121 is provided in the base 122. In the present embodiment, the electroencephalogram electrode member 120 further includes a conductive member 124, a wiring line 127, and a cover 129.

The conductive member 124 is, for example, a metal, and has a first portion 124a and a second portion 124b. The first portion 124a and the second portion 124b are integrally formed.

The first portion 124a has a tubular shape. The through hole provided in the conductive member 124 and the through hole provided in the base 122 communicate with each other, and the third through hole 121 is formed by these through holes. A screw groove is provided on an outside of the first portion 124a.

The second portion 124b has a plate shape. In the example of FIG. 13, the cover 129 covers a part of the conductive member 124 and a part of the base 122. The cover 129 is formed of, for example, a resin, and has insulating properties. The base 122 is fixed to a main surface of the second portion 124b.

The protruding portion 123 has a first portion 123a, a conductive portion 123b, and a second portion 123c. A plurality of protruding portions 123 are provided on a surface of the base 122 on a side opposite to the conductive member 124 side. The base 122 and the first portion 123a are integrally provided by a rubber-like elastic body. 10 or more of the protruding portions 123 may be provided. A shape of the first portion 123a is, for example, a cone or a pyramid such as a polygonal pyramid. The conductive portion 123b is provided to cover the first portion 123a. In addition, a distal end of the first portion 123a is covered with the second portion 123c. The second portion 123c is a member having a spherical shape of a gel-like material (also referred to as a hydrogel) containing moisture in the inside, and is attached to be inserted into a distal end part of the first portion 123a.

In a case where the electroencephalogram electrode member 120 is pressed against the head 20 for the electroencephalogram measurement, the second portion 123c comes into contact with the head 20. In this case, an electrolyte (in general, salt content) of the scalp 22 is taken into the second portion 123c. As a result, the electroencephalogram electrode member 120 and the scalp 22 are electrically conductive. A shape of the second portion 123c is not limited to the sphere. In addition, the gel-like material constituting the second portion 123c is not particularly limited as long as it can have sufficient moisture and can achieve sufficient strength and flexibility in a case of being pressed against the head 20; and for example, an acrylic hydrogel or a silicone hydrogel can be used.

A material of the base 122 and the first portion 123a will be described. The base 122 and the first portion 123a are configured to have a rubber-like elastic body. Specifically, the rubber-like elastic body is a rubber or a thermoplastic elastomer (also simply referred to as "elastomer (TPE)"). Examples of the rubber include a silicone rubber. Examples of the thermoplastic elastomer include styrene-based TPE (TPS), olefin-based TPE (TPO), vinyl chloride-based TPE (TPVC), urethane-based TPE (TPU), ester-based TPE (TPEE), and amidebased TPE (TPAE).

The conductive portion 123b is formed of, for example, a paste containing a highly conductive metal. The conductive portion 123b contains one or more selected from the group consisting of copper, silver, gold, nickel, tin, lead, zinc, bismuth, antimony, and an alloy thereof.

A wiring line 127 connected to the conductive portion 123b is provided inside the first portion 123a. The wiring line 127 electrically connects the conductive portion 123b and the conductive member 124. The wiring line 127 may be configured of, for example, a conductive fiber. As the conductive fiber, one or more selected from the group consisting of metal fiber, metal-coated fiber, carbon fiber, conductive polymer fiber, conductive polymer-coated fiber, and conductive paste-coated fiber can be used. These may be used alone or in combination of two or more thereof.

With reference to FIG. 8, the holding member 140 and the tube 151 will be described. In the present embodiment, an outer shape of the holding member 140 is substantially rod-like. A through hole is provided inside the holding member 140. The through hole of the holding member 140 connects the inside and the outside of the support 110 in a state in which the holding member 140 is attached to the support 110.

The electroencephalogram electrode member 120 is attached to the conductive portion 164 (that is, attached to the elastic member 130) by screwing the first portion 124a of the conductive member 124 into the conductive portion 164. In a state in which the electroencephalogram electrode member 120 is attached to the conductive portion 164, the first portion 124a penetrates the conductive portion 164, and a distal end of the first portion 124a protrudes to the outside of the conductive portion 164. A screw groove is provided inside the one end 141 of the holding member 140. The holding member 140 can hold the electroencephalogram electrode member 120 by screwing the screw groove of the one end 141 of the holding member 140 and the portion of the first portion 124a which protrudes from the conductive portion 164. The operator can attach or detach the holding member 140 to or from the electroencephalogram electrode member 120 by holding the other end 142 of the holding member 140 and rotating the holding member 140 around the z direction.

A connection portion for attaching the injection member 152 is provided at the other end 142 of the holding member 140. The connection portion is a part of the through hole of the holding member 140, and is configured to be able to fit the connecting member 153. The injection member 152 is, for example, a syringe, and has a liquid container portion. The tube 151 is, for example, made of a metal. In a state in which the connecting member 153 and the tube 151 are attached to the injection member 152, the tube 151 is inserted into the third through hole 121, and the connecting member 153 is fitted into the connection portion of the holding member 140. In this manner, in a state in which the tube 151 is inserted into the through hole of the holding member 140 and the third through hole 121 of the electroencephalogram electrode member 120, the injection member 152 and the tube 151 are fixed to the holding member 140 through the connecting member 153.

In a state in which the tube 151 is inserted into the third through hole 121 of the electroencephalogram electrode member 120, the tube 151 penetrates the base 122. The liquid pushed out from the injection member 152 is supplied to the scalp 22 through the tube 151.

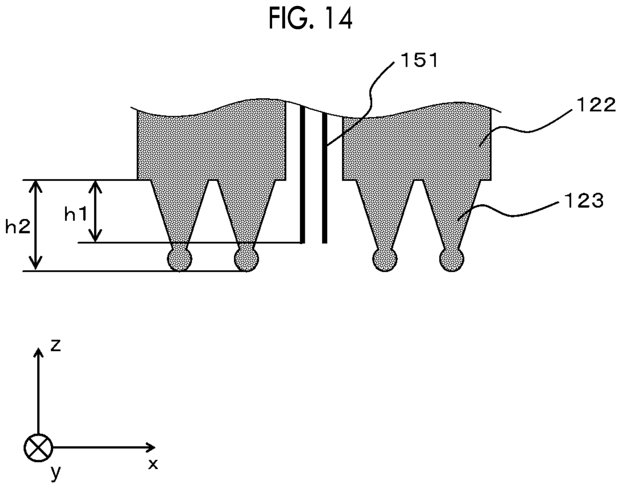

FIG. 14 is a view showing a relationship between the protruding portion 123 and the tube 151. In a state in which the tube 151 is inserted into the third through hole 121 of the electroencephalogram electrode member 120, a protrusion height h1 of the tube 151 from the base 122 is smaller than a protrusion height h2 of the protruding portion 123 from the base 122. In this manner, a distal end of the tube 151 is prevented from touching the scalp 22 and causing damage.

With reference to FIGS. 3, 5, 9, and 13, an electrical connection relationship in the electroencephalogram measurement device 10 will be described below. The electroencephalogram measurement device 10 further includes a wiring line 163, a circuit 162, a wiring line 165, a signal processing unit 160, and a wiring line 161 for reference potential measurement. Among the above, the conductive portion 164, the wiring line 163, the circuit 162, and the wiring line 165 are provided for each electroencephalogram electrode member 120. The wiring line 163 and the circuit 162 are fixed to the elastic member 130 together with the conductive portion 164.

In a case where the scalp 22 comes into contact with the second portion 123c, an electrical signal from the scalp 22 is transmitted to the conductive member 124 through the second portion 123c, the conductive portion 123b, and the wiring line 127. The electrical signal obtained by each of the electroencephalogram electrode members 120 is transmitted from the conductive member 124 of the electroencephalogram electrode member 120 to the signal processing unit 160 through the conductive portion 164, the wiring line 163, the circuit 162, and the wiring line 165. The circuit 162 includes, for example, a preamplifier which amplifies the electrical signal from the electroencephalogram electrode member 120. The signal processing unit 160 acquires the electrical signal from the plurality of the electroencephalogram electrode members 120 (from the circuit 162). The signal processing unit 160 performs, for example, processing such as amplification of the electrical signal of the electroencephalogram, analog-to-digital conversion, and frequency filtering. In addition, the signal processing unit 160 can record the electroencephalogram signal data obtained by performing the processing in a recording unit provided in the signal processing unit 160. In addition, the signal processing unit 160 can transmit the electroencephalogram signal data to an external device by wired communication or wireless communication. The signal processing unit 160 is implemented by, for example, an integrated circuit. It is preferable that the signal processing unit 160 has a built-in battery. In this manner, it is not necessary to connect a power supply line to the signal processing unit 160 for power supply. Accordingly, the subject to be measured can move or act to some extent freely during the measurement. In addition, noise depending on the frequency of the power supply can be prevented. The wiring line 161 for reference potential measurement connects the signal processing unit 160 and a reference electrode (not shown) to each other. The reference electrode is an electrode for acquiring a reference potential which is a reference in the measurement of the electroencephalogram signal. The reference electrode acquires the reference potential by, for example, being attached to a lower part of an earlobe or an upper part of an outer ear with a clip or being attached to a bone of a back surface of the outer ear.

Next, an operation and effect of the present embodiment will be described. According to the present embodiment, the electroencephalogram measurement device 10 includes the support 110, the electroencephalogram electrode member 120, and the tube 151. The support 110 is mountable on the head 20. The electroencephalogram electrode member 120 is held by the support 110. The tube 151 passes through the third through hole 121 provided in the electroencephalogram electrode member 120. Therefore, the liquid can be supplied to the scalp to be easily measured.

Hereinabove, the embodiments of the present invention have been described with reference to the drawings. However, the embodiment is merely an example of the present invention, and various configurations other than the above-described configurations can also be adopted.

Priority is claimed on Japanese Patent Application No. 2023-104854, filed on June 27, 2023, the disclosure of which is incorporated herein by reference.

### REFERENCE SIGNS LIST

- 10: electroencephalogram measurement device
- 20: head
- 22: scalp
- 110: support
- 111: substrate
- 112: covering member
- 114: second through hole
- 120: electroencephalogram electrode member
- 121: third through hole
- 122: base
- 123: protruding portion
- 124: conductive member
- 127: wiring line
- 129: cover
- 130: elastic member
- 131: first through hole
- 140: holding member
- 151: tube
- 152: injection member
- 153: connecting member
- 160: signal processing unit
- 161: wiring line for reference potential measurement
- 162: circuit
- 163, 165: wiring line
- 164: conductive portion
- 170: belt

## Claims

1. An electroencephalogram measurement apparatus comprising:
a support which is mountable on a head;
an electroencephalogram electrode member which is held by the support; and
a tube which passes through a through hole provided in the electroencephalogram electrode member.

2. The electroencephalogram measurement apparatus according to Claim 1,
wherein the electroencephalogram electrode member includes a base and one or more protruding portions provided in the base, and
the through hole is provided in the base.

3. The electroencephalogram measurement apparatus according to Claim 2,
wherein the tube penetrates the base.

4. The electroencephalogram measurement apparatus according to Claim 3,
wherein a protrusion height of the tube from the base is smaller than a protrusion height of the protruding portion from the base.

5. The electroencephalogram measurement apparatus according to any one of Claims 1 to 4,
wherein the tube is insertable into and removable from the through hole of the electroencephalogram electrode member.

6. The electroencephalogram measurement apparatus according to any one of Claims 1 to 5, further comprising:
an injection member for injecting a liquid into the tube.
